(19) Europäisches Patentamt
European Patent Office
Office européen des brevets

(11) **EP 2 636 362 A1**

(12) **EUROPEAN PATENT APPLICATION**

(43) Date of publication:
**11.09.2013 Bulletin 2013/37**

(51) Int Cl.:
**A61B 5/00** *(2006.01)*

(21) Application number: **13157944.3**

(22) Date of filing: **06.03.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **08.03.2012 JP 2012051616**

(71) Applicant: **Canon Kabushiki Kaisha**
**Tokyo 146-8501 (JP)**

(72) Inventor: **Tanaka, Takatoshi**
**Tokyo, Tokyo 146-8501 (JP)**

(74) Representative: **TBK**
**Bavariaring 4-6**
**80336 München (DE)**

(54) **Object information acquiring apparatus**

(57)     The present invention employs an object information acquiring apparatus that includes a plate configured to hold an object, a probe unit including a probe configured to receive an acoustic wave generated from the object, a tank configured to store matching liquid for acoustically matching the plate and the probe, a supplying unit configured to supply the matching liquid in the tank to a space between the probe unit and the plate, and a generating unit configured to generate property information of an inside of the object on the basis of the acoustic wave. The tank includes an aperture in a portion facing the plate, and the volume of the tank is larger than a sum of the internal volume of the supplying unit and the volume of the space between the plate and the probe unit.

FIG. 1

## Description

BACKGROUND OF THE INVENTION

Field of the Invention

The present invention relates to an object information acquiring apparatus.

Description of the Related Art

As a diagnostic apparatus that makes use of an acoustic wave, there has been known an intravital observation apparatus that irradiates an acoustic wave on an object and converts a reflected wave (echo) of the acoustic wave into a video to pickup an image of the inside of the object. In such an intravital observation apparatus, an acoustic wave probe that oscillates an acoustic wave and receives a reflected wave (echo) of the acoustic wave to output a signal is used. The intravital observation apparatus converts the acoustic wave received by the acoustic wave probe into an electric signal and generates and displays an image.

Recently, the development of a diagnostic system of photoacoustic tomography (PAT) that makes use of a photoacoustic effect is in progress. The diagnostic system irradiates illumination light (a near infrared ray) from a pulsed light source such as an Nd:YAG laser on an object. The diagnostic system receives, with a probe, an acoustic wave generated by the photoacoustic effect on the inside of the object when the light is irradiated and generates and displays an image.

In such an apparatus that measures an acoustic wave generated from the inside of a living organism, it is necessary to match acoustic impedance in order to reduce a loss of the acoustic wave from the inside of the living organism. In particular, the apparatus has to be configured such that the air does not intervene between members to which the acoustic wave is transmitted.

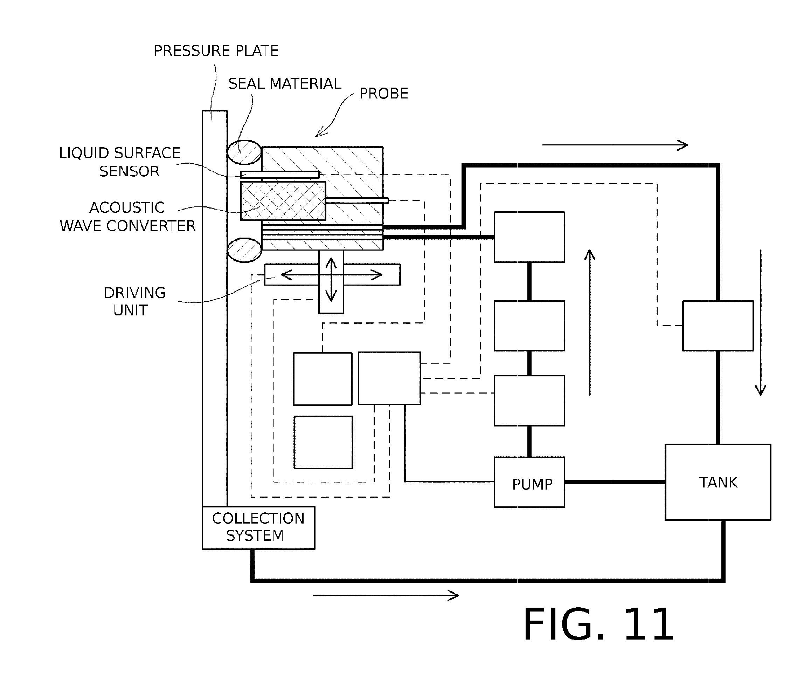

As an example of such an apparatus, there is an acoustic wave measuring apparatus disclosed in Japanese Patent Application Laid-Open No. 2011-103913 (Patent Literature 1). A schematic diagram of the acoustic wave measuring apparatus is shown in Fig. 11. In Fig. 11, matching liquid filled in a tank is supplied to a probe through a pipe by a pump functioning as a supplying unit. During measurement, a space between a pressuring plate and the probe is filled with the matching liquid. A collection system functioning as a collecting unit for collecting the matching liquid is provided in a lower part of the pressuring plate. The collection system includes a receiving container (a collection tray) that receives the matching liquid flowing down along the pressuring plate and a pipe for connecting the receiving container and the tank. The matching liquid leaking from a probe unit is collected by the collection system and circulated to the tank.

Fig. 12 shows the structure of the probe disclosed in Japanese Patent Application Laid-Open No. 2011-103913. The probe includes an acoustic wave converter that converts an acoustic wave into an electric signal, a housing that houses the acoustic wave converter, and a seal material for the matching liquid. The matching liquid is held between the housing and the pressuring plate to match acoustic impedance. These components are collectively referred to as acoustic wave probe unit as well. The seal material is provided on a measurement surface of the acoustic wave probe unit to surround the periphery of the acoustic wave converter. A delivery port for supplying the matching liquid and a collection port for collecting the matching liquid are provided in a lower part of a space surrounded by the seal material. An opening for allowing the air mixed in the matching liquid to escape is formed in an upper part of the seal material.

Patent Literature 1: Japanese Patent Application Laid-Open No. 2011-103913

SUMMARY OF THE INVENTION

In the acoustic wave measuring apparatus adopting the configuration of the related art, the acoustic impedance is matched by circulating the matching liquid in the tank through the apparatus using a circulation system such as a pump and a pipe and supplying the matching liquid to between the pressuring plate and the probe. However, when some abnormality occurs in the circulation system, it is likely that a deficiency occurs in the supply of the matching liquid and eventually affects adversely acquisition of an image of the inside of an object.

As one of abnormalities that could occur in the circulation system, there is an overflow from the receiving container (the collection tray), which is the collection system that receives the matching liquid flowing down along the pressuring plate. In the conventional configuration, the collection system and the tank are separate. That is, if the tank is removed, it is highly likely that a total amount of the matching liquid circulating through the apparatus exceeds a total amount of the matching liquid that could be stored by the collection system. Therefore, when the volume of the collection tray is smaller than the volume of the tank, it is likely that the matching liquid overflows the tray when the pipe is clogged up in the collection system. In other words, in the conventional configuration, it is not taken into account to prevent the matching liquid from overflowing the collection tray.

As another abnormality that could occur in the circulation system, there is a liquid leak of the matching liquid that could be caused by, for example, a failure of the pipe of the collection system. In the conventional configuration, even if the liquid leak occurs and the matching liquid in the tank is in short supply, control for stopping a supply system is not performed. Therefore, the leak of the matching liquid cannot be automatically detected, and the matching liquid continues to leak until a user visually finds the leak.

The present invention has been devised in view of the problem. The present invention is developed to provide a technique for coping with an abnormality in the circulation of the matching liquid when the matching liquid used for the probe, which acquires an acoustic wave, is collected and circulated.

The present invention in its first aspect provides an object information acquiring apparatus as specified in claims 1 to 8.

According to the apparatus according to the present invention, it is possible to provide a technique for coping with an abnormality in the circulation of the matching liquid when the matching liquid used for the probe, which acquires an acoustic wave, is collected and circulated.

Further features of the present invention will become apparent from the following description of exemplary embodiments with reference to the attached drawings.

BRIEF DESCRIPTION OF THE DRAWINGS

Fig. 1 is a block diagram of an acoustic wave measuring apparatus according to a first embodiment;
Figs. 2A and 2B are perspective views of an acoustic wave measuring apparatus according to the present invention;
Fig. 3 is a perspective view of a tank for matching liquid;
Fig. 4 is a sectional view of the tank for matching liquid;
Fig. 5 is a flowchart for explaining pre-measurement preparation processing in the first embodiment;
Fig. 6 is a flowchart for explaining monitoring processing for matching liquid during measurement in the first embodiment;
Fig. 7 is a track diagram of an acoustic wave probe unit during measurement;
Fig. 8 is a block diagram of an acoustic wave measuring apparatus according to a second embodiment;
Fig. 9 is a flowchart for explaining pre-measurement preparation processing in the second embodiment;
Fig. 10 is a flowchart for explaining monitoring processing for matching liquid during measurement in the second embodiment;
Fig. 11 is a block diagram of a conventional acoustic wave measuring apparatus; and

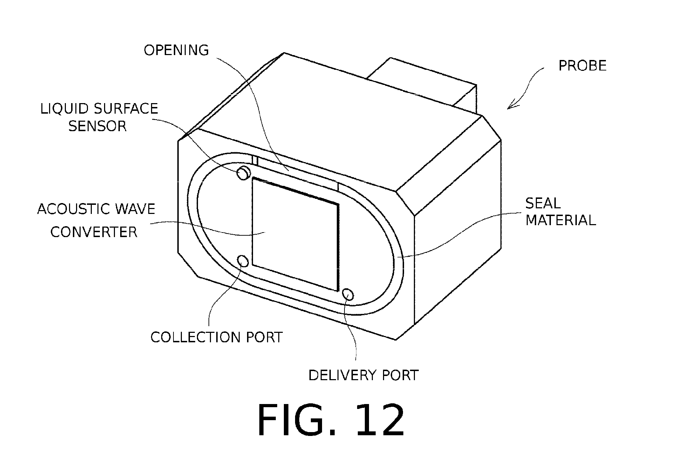

Fig. 12 is a perspective view of a conventional acoustic wave probe.

DESCRIPTION OF THE EMBODIMENTS

<First Embodiment>

Preferred embodiments of the present invention are explained below with reference to the drawings. However, dimensions, materials, and shapes of components explained below, relative arrangement of the components, and the like should be changed as appropriate according to the configuration of an apparatus to which the present invention is applied and various conditions and are not meant to limit the scope of the present invention to the below description.

An acoustic wave measuring apparatus according to the present invention is an apparatus used for receiving an acoustic wave generated from the inside of a living organism, converting the acoustic wave into an electric signal, and acquiring property information (a tomogram image, an optical feature value distribution, etc.) of the inside of the living organism from the signal. Such property information is referred to as object information as well. Therefore, the acoustic wave measuring apparatus according to the present invention can be referred to as object information acquiring apparatus as well. The acoustic wave measuring apparatus can be applied to an intravital observation apparatus such as an ultrasound diagnostic apparatus or a photoacoustic tomography apparatus (PAT) that makes use of ultrasonic echo. The configuration of a tank for collecting and filling matching liquid is mainly explained. As the acoustic wave, typically, an ultrasound wave is used. However, an elastic wave in an audible range having a frequency lower than the frequency of the ultrasound wave may be used.

(Acoustic wave measuring apparatus)

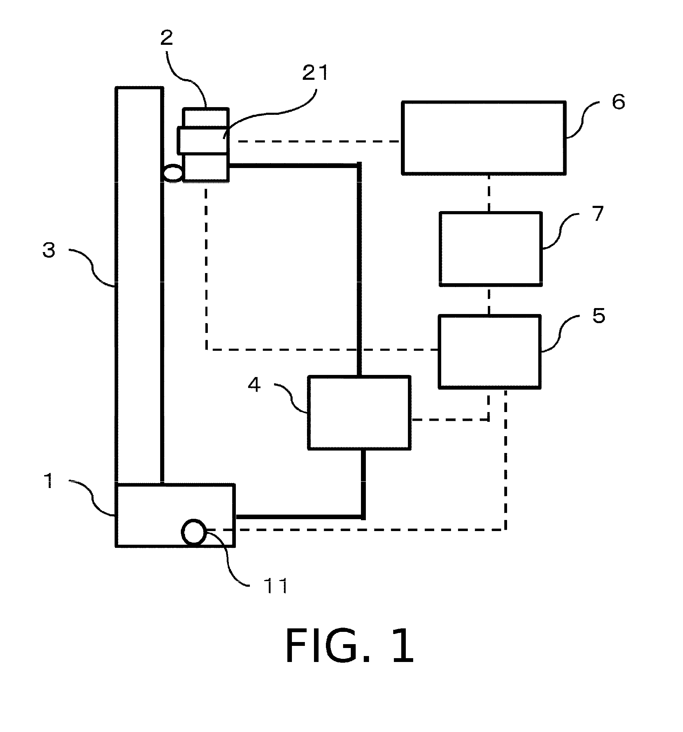

Fig. 1 is a block diagram of an acoustic wave measuring apparatus according to the first embodiment. The acoustic wave measuring apparatus includes, as basic components, a tank 1, a probe unit 2 including a probe 21, a pressuring plate 3 that pressuring and holding an object, and a pump 4 that delivers and circulates matching liquid. Further, the acoustic wave measuring apparatus according to this embodiment includes a control unit 5, an image generating unit 6, and a display unit 7.

The tank 1 is arranged in a position below the pressuring plate 3. The position below the plate is typically a position vertically below the plate 3. However, the position of the tank 1 is not limited to the position vertically below the plate 3 as long as the matching liquid overflowing the probe unit 2 flows into the tank 1 from above. The pressuring plate 3 and the tank 1 are coupled. The tank 1 includes an aperture in a portion facing the pressuring plate 3. The matching liquid stored in the tank 1 is delivered by the pump 4 (referred to as matching liquid supply pump as well) functioning as a supplying unit and supplied to the probe unit 2 through a pipe. During measurement, a space between the pressuring plate 3 and the probe 21 is filled with the matching liquid. The probe unit 2 moves along the pressuring plate 3. The probe unit 2 moves while causing the matching liquid to overflow. The overflowed matching liquid flows down along the pressuring plate 3 to be collected in the tank 1.

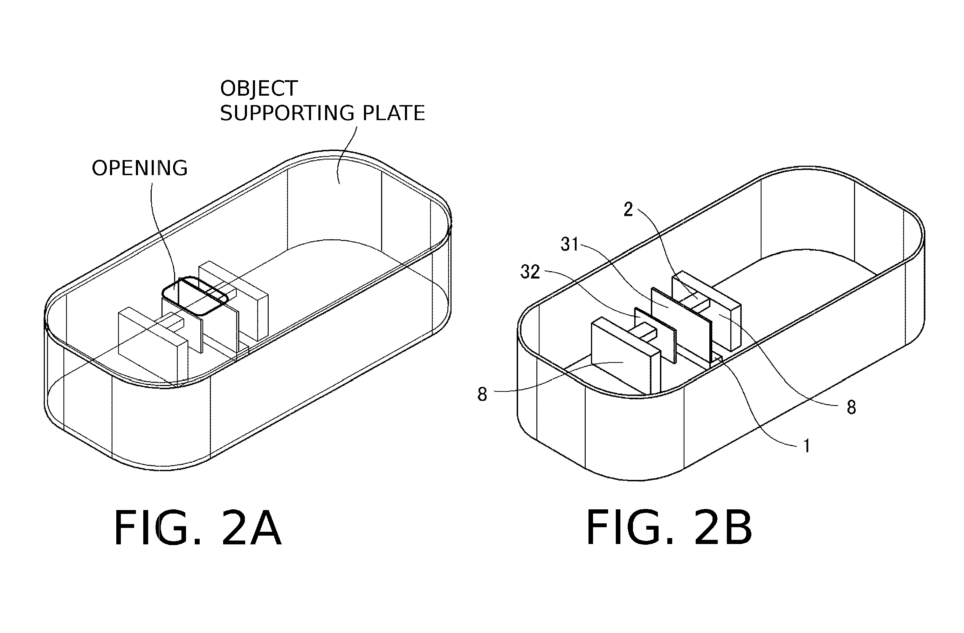

Figs. 2A and 2B are perspective views of an acoustic wave measuring apparatus according to the present invention. As shown in Fig. 2A, the acoustic wave measuring apparatus includes a lid-like object supporting plate, i.e., an object supporting table for supporting an object. Fig. 2A is a diagram showing a positional relation between the object supporting plate and an internal structure of the acoustic wave measuring apparatus seeing through the object supporting plate. Fig. 2B shows a state in which the object supporting plate is removed. As shown in Fig. 2B, the pressuring plate 3 shown in Fig. 1 is one of two pressuring plates 31 and 32 arranged to be parallel to each other. It is possible to pressure and hold the object by adjusting a space between the pressuring plates 31 and 32.

However, the present invention is not limited to a method of placing an examinee in a prone position on the supporting table, causing the examinee to hang down a breast from between the two pressuring plates, and pressing and holding the breast. For example, a method of placing the examinee in an upright position and holding the breast from top and bottom with the two pressuring plates may be adopted. In this case, a tank and the like of a collection system are arranged in a position below, preferably, vertically below the pressuring plate on the lower side to collect the matching liquid flowing to the collection system with the gravity or through a channel. A pressuring method is not limited to the holding by the two pressuring plates. A method of pressing the breast against one plate may be adopted.

A mechanism (not shown) for adjusting the space between the pressuring plates may use either an electric motor or manual operation of a user as a power source. When measurement is performed using the photoacoustic effect, a light source that irradiates pulsed light is attached to stages 8. The two stages 8 are synchronously controlled to synchronously move the light source and the probe unit 2 arranged to be opposed to each other across the object. An acoustic wave generated in the object by the light emitted from the light source is measured by the probe unit 2.

(Tank)

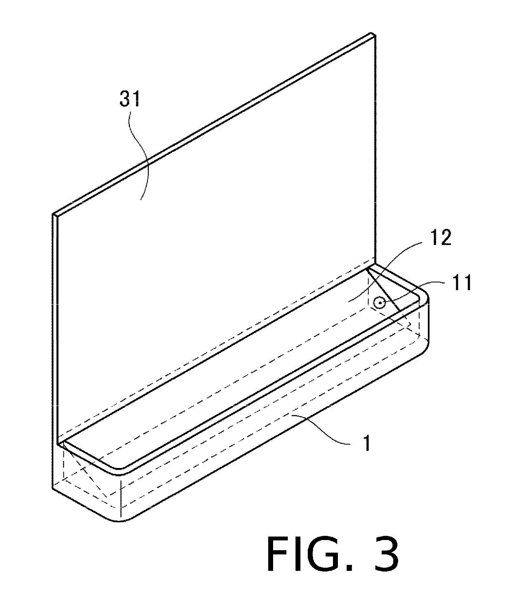

Fig. 3 is a perspective view of the tank 1 functioning as a container for matching liquid. The tank 1 desirably always stores the matching liquid on the inside thereof both before measurement and during measurement. However, in a state in which the matching liquid circulates to be always supplied to the probe unit 2, the matching liquid is sufficient for realizing acoustic matching. Therefore, an amount of the matching liquid stored in the tank 1 does not need to be large.

The matching liquid acoustically matches the probe and the pressuring plates. The tank 1 includes a matching-liquid-surface detecting unit 11 that detects whether a matching liquid surface is present above a predetermined position. The height of the matching-liquid-surface detecting unit 11 in the tank 1 is a predetermined value set according to an amount of the matching liquid circulating through the apparatus, a path of the circulation, and a required time of the circulation. When the matching liquid surface in the tank 1 is below a detection position, it is likely that the matching liquid is leaking in the apparatus. Therefore, when the matching liquid surface in the tank is below the detection position, the matching liquid supply pump functioning as the supplying unit for the matching liquid is controlled. For example, the circulation of the matching liquid is stopped. By adopting such a configuration, among abnormalities of the circulation system that are problems to be solved by the present invention, it is possible to detect a leak of the matching liquid and suppress contamination in the apparatus and a failure of the apparatus.

An exclusive liquid surface sensor is provided in the matching-liquid-surface detecting unit 11. As the liquid surface sensor, a sensor of any type such as an optical type, an ultrasonic type, or a capacitance type may be used.

The volume of the tank 1 that can store the matching liquid is desirably designed as indicated by Expression (1) below. The volume of the tank 1 is represented as V, the volume of portions filled with the matching liquid other than the tank 1 is represented as V1, the volume of lubricant in the tank 1 in which the matching liquid surface reaches liquid surface height detected by the matching-liquid-surface detecting unit 11 is represented as V2, and the volume of the lubricant that evaporates before replacement of the lubricant is represented as V3.

$$V>V1+V2+V3 \quad (1)$$

The portions other than the tank 1 is, for example, the space between the probe unit and the pressuring plate, a channel on the pressuring plate, the pump, and pipes that connect the probe unit, the pressuring plate, and the pump. V1 is considered to be a sum of internal volumes of the portions other than the tank.

Expression (1) indicates that the tank does not overflow even if all the matching liquid circulating through the apparatus flows into the tank. As long as an expression for estimation of the tank volume takes into account this indication, the tank volume does not always have to be estimated according to Expression (1). For example, the matching liquid is mainly present in, other than the tank, the circulation system (the supplying unit) such as the pump and the pipes and the sealed space between the probe unit and the pressuring plate. Therefore, if the internal volume of the supplying unit and the volume of the sealed space are added up, a necessary volume of the tank can be estimated. As the volume V3 of the evaporating lubricant, a pseudo value may be used.

By designing the volume as explained above, even if the pipes or the like are clogged up, since the volume of the tank 1 is sufficiently large, it is possible to prevent the lubricant from overflowing the tank 1. By adopting such a configuration, it is possible to cope with the overflow of the tank among the abnormalities of the circulation system that are the problems to be solved by the present invention.

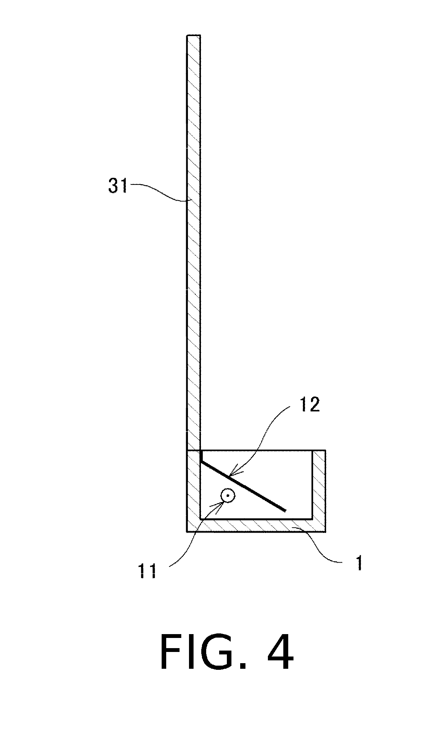

Fig. 4 is a sectional view of the tank 1. A cover 12 is set to surround an inner side wall surface of the tank 1 on the pressuring plate 3 side to a region above the matching-liquid-surface detecting unit 11. The cover 12 covers the region above the matching-liquid-surface detecting unit 11.

The cover 12 suppresses a situation in which the liquid surface in the tank 1 is disturbed by droplets of the matching liquid flowing down from the probe unit 2 or the pressuring plate 3 and the liquid surface sensor misdetects the liquid surface. The cover 12 is desirably arranged to be tilted obliquely downward viewed from the pressuring plate 3. This is for the purpose of preventing the flowing-down droplets from scattering and contaminating the inside of the apparatus when hitting the cover 12.

(Measuring and controlling method)

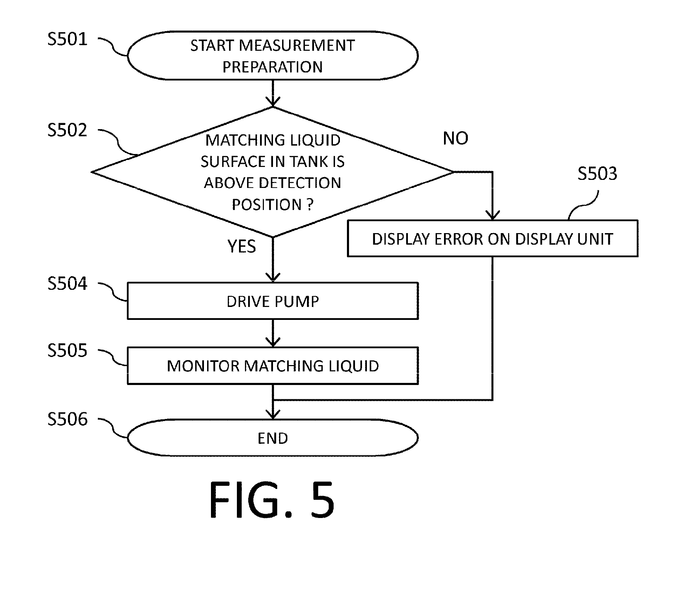

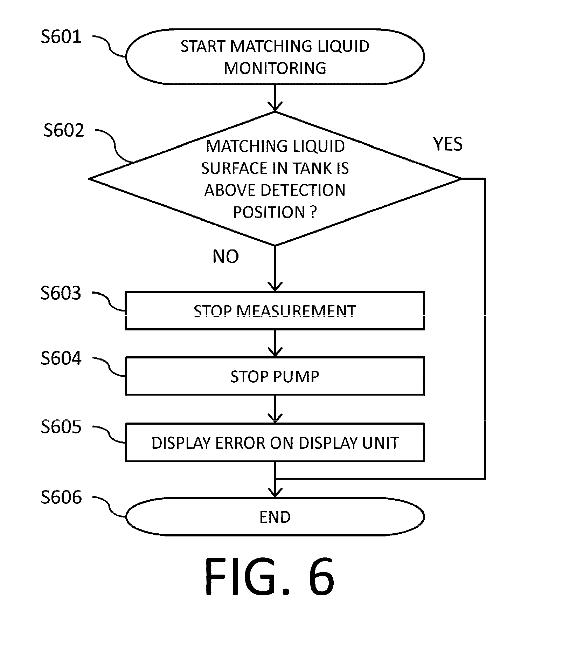

A measuring and controlling method by the apparatus according to this embodiment is explained with reference to Figs. 5 and 6. Fig. 5 is a flowchart for explaining a flow of pre-measurement preparation processing in this embodiment. Fig. 6 is a flowchart for explaining a flow of monitoring processing for the matching liquid during measurement in this embodiment. The processing explained below is executed by the control unit 5 shown in Fig. 1. The control unit 5 includes a microcontroller or a computer and performs control of the pump and the like.

In step S501 in Fig. 5, pre-measurement processing is started.

In step S502, first, the control unit 5 confirms, on the basis of a detection result of the matching-liquid-surface detecting unit 11, whether the matching liquid surface in the tank 1 is above a liquid surface detection position.

When it is confirmed that the matching liquid surface in the tank 1 is above the liquid surface detection position (YES in S502), the control unit 5 proceeds to step S504 and drives the pump 4 functioning as the supplying unit. Subsequently, the control unit 5 proceeds to step S505 and performs matching liquid monitoring processing. This processing is processing for abnormality detection for the circulation system that can be performed not only at a measurement preparation stage but also periodically even during measurement or according to an instruction of the user.

On the other hand, when it is confirmed that the matching liquid surface in the tank 1 is below the liquid surface detection position (NO in S502), the control unit 5 causes the display unit 7 to display an error (S503). The user who confirms the error checks whether the matching liquid leaks in the apparatus and, after taking measures according to necessity, fills the matching liquid and performs the pre-measurement preparation again.

After the processing, the control unit 5 proceeds to step S506 and ends the pre-measurement preparation.

The control unit 5 monitors whether the liquid surface of the matching liquid in the tank 1 is above a detection position of the matching-liquid-surface detecting unit 11 even during measurement. Specifically, the control unit 5 performs processing shown in Fig. 6.

In step S601, the control unit 5 starts matching liquid monitoring.

Subsequently, in step S602, the control unit 5 compares the matching liquid surface and a liquid surface detection position.

The control unit 5 continuously performs measurement while it is confirmed that the matching liquid surface is above the detection position. That is, when the matching liquid surface is above the liquid surface detection position (YES in S602), the control unit 5 proceeds to step S606 and ends the processing. Thereafter, the control unit 5 starts the monitoring processing again after a fixed time.

However, when it is detected that the matching liquid surface is below the detection position (NO in S602), the control unit 5 stops the measurement in step S603. Consequently, it is possible to prevent an inaccurate image from being acquired in a state in which a supply abnormality of the matching liquid occurs. Further, in step S604, the control unit 5 stops the pump 4 functioning as the supplying unit.

In step S605, the control unit 5 displays an error on the display unit 7. The user who confirms the error display checks whether the matching liquid leaks in the apparatus, and after taking measures according to necessity, fills the matching liquid and performs the pre-measurement preparation again.

Finally, in step S606, the control unit 5 ends the monitoring processing. By monitoring an amount of the matching liquid in the tank 1, it is possible to quickly detect a matching liquid leak in the apparatus.

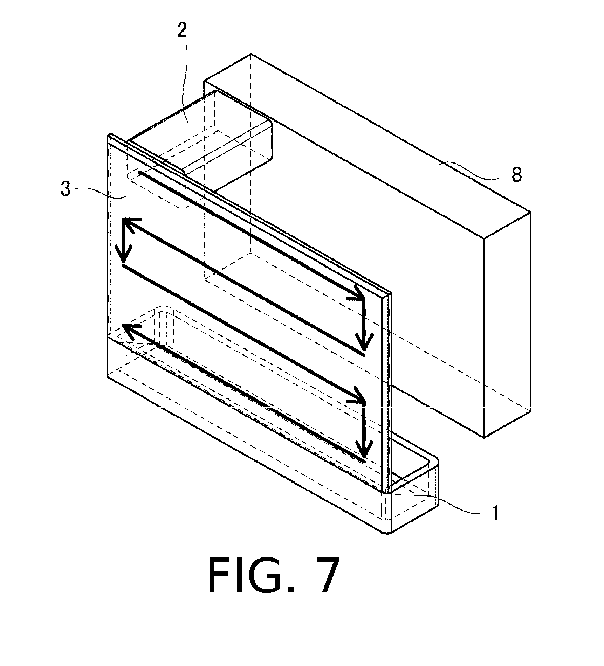

Fig. 7 shows a track of movement of the acoustic wave probe unit 2 during measurement. The probe unit 2 is arranged in an initial position on the stage 8 during the pre-measurement preparation. In this figure, the probe unit 2 is arranged at the top side end of a measurement range during the initial measurement start. When the measurement is started, the probe unit 2 moves in the horizontal direction. Upon reaching the side end on the opposite side, the probe unit 2 moves downward and moves in the horizontal direction again. The probe unit 2 repeats this action until the probe unit 2 moves through the entire measurement range. The probe unit 2 moves while causing the matching liquid to overflow. The matching liquid continues to be always supplied to the probe unit 2 by the supplying unit. The probe unit 2 returns to the initial position after moving through the entire measurement range.

The initial position of the probe unit 2 is a position where the probe unit 2 retracts from a portion for pressuring the object such that the pressured object can be checked through the pressuring plate 3. For example, the initial position is set in an obliquely downward position of the pressuring plate 3 to prevent the object and the probe unit 2 from overlapping viewed from the back of the pressuring plate 3. It is possible to set a camera behind the pressuring plate 3 to photograph the object, displays an image on the display unit 7 and checks a photographed portion, and designate a portion desired to be photographed. The movement of the probe unit 2 can be performed by, for example, a mechanical moving unit that can move in the lateral direction and the longitudinal direction.

The probe 21 converts a received acoustic wave into an electric signal and outputs the electric signal to the image generating unit 6. The image generating unit 6 converts information concerning the inside of the object into an image on the basis of the signal. As a method of the conversion into an image, a conventional method can be used. For example, the information can be converted into an image by various image reconfiguring methods. The image converted from the information is displayed on the display unit 7. The user can check the image.

With the configuration of this embodiment explained above, it is possible to cope with an abnormality of the circulation system for the matching liquid. That is, it is possible to prevent the matching liquid from overflowing the collection tray and, if a leak of the matching liquid occurs, detect the leak and suppress contamination of the apparatus. It is possible to acquire a satisfactory image of the object by detecting an abnormality and feeding back the abnormality in this way.

The prevention of a leak of the matching liquid by setting the volume of the tank equal to or larger than the sum of the internal volume of the supplying unit and the volume of the sealed space and the detection of a liquid leak by the detection of the liquid surface in the tank are not always simultaneously performed.

<Second Embodiment>

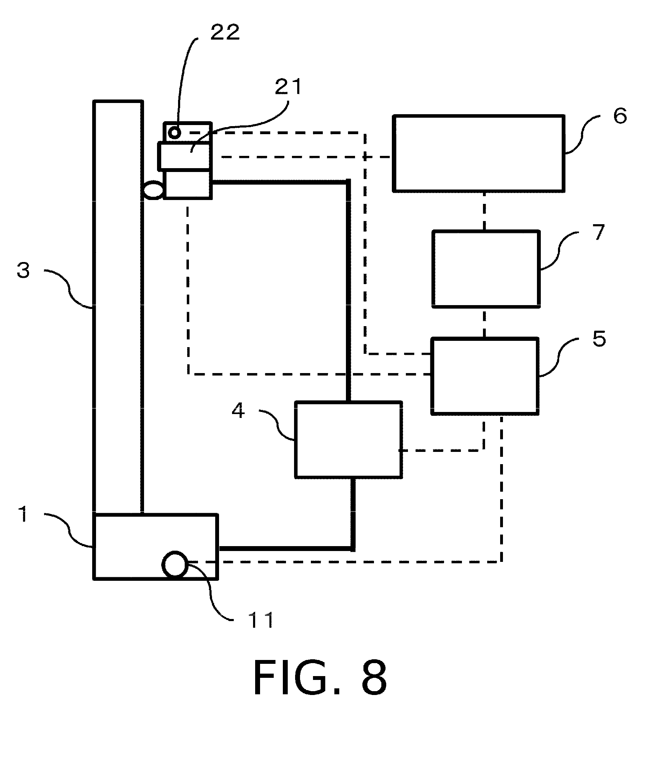

An acoustic wave measuring apparatus according to a second embodiment is explained with reference to Fig. 8. Fig. 8 is a block diagram of the acoustic wave measuring apparatus according to this embodiment. The acoustic wave measuring apparatus according to this embodiment includes a matching-liquid detecting unit 22 (in this specification, referred to as a second detecting unit as well) that detects whether the matching liquid is sufficiently filled in the space between the probe 21 and the pressuring plate 3. The matching-liquid detecting unit 22 is controlled by the control unit 5. With such a configuration, it is possible to detect a failure in supply of the matching liquid into a probe unit and provide the acoustic wave measuring apparatus having high reliability.

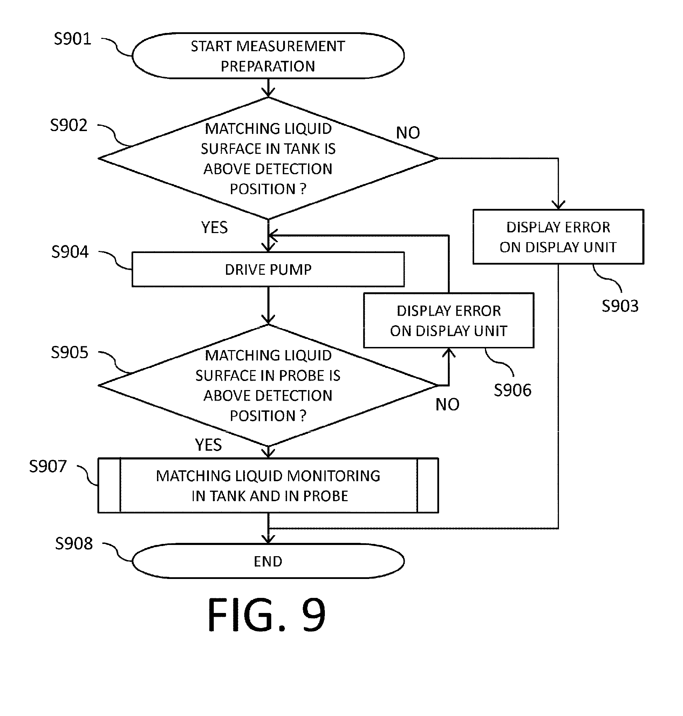

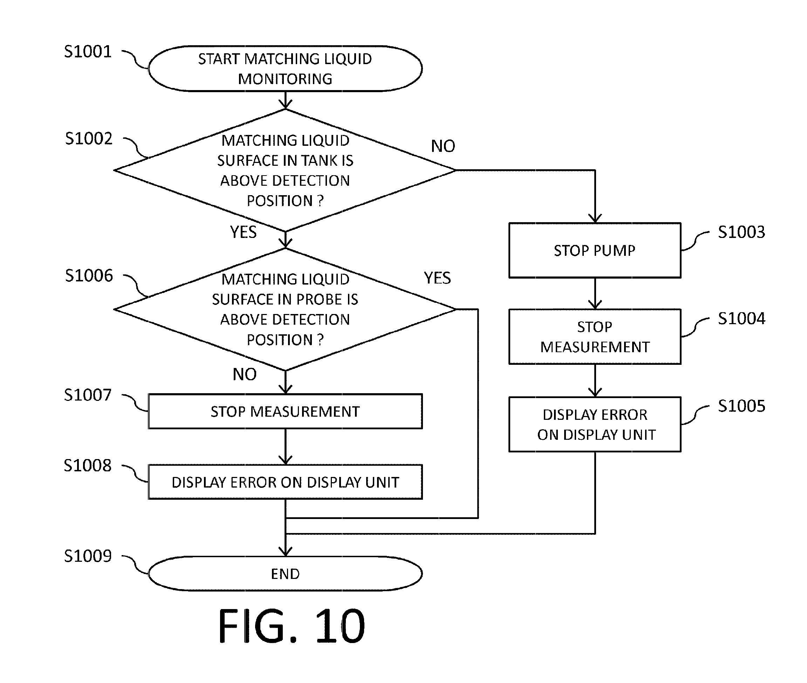

A measuring and controlling method by the apparatus according to this embodiment is explained with reference to Figs. 9 and 10. Fig. 9 is a flowchart for explaining a flow of pre-measurement preparation processing. Fig. 10 is a flowchart for explaining a flow of detection processing for the matching liquid during measurement. Differences of the flows from the flows shown in Figs. 5 and 6 are mainly explained.

In Fig. 9, the start of measurement preparation in step S901 to detection of the liquid surface of the matching liquid in the tank 1 in step S902, error display in step S903, and pump driving in step S904 are the same as steps S501 to S504 shown in Fig. 5. At this point, concerning whether an amount of the matching liquid is sufficient, a detection point may be provided at predetermined height in the probe unit and, if the matching liquid is present above the detection point, it may be determined that a second predetermined amount of the matching liquid is filled.

In step S905, the control unit 5 detects, using the matching-liquid detecting unit 22 in the probe unit, whether the liquid surface of the matching liquid in a sealed space in the probe 21 is above a detection position. When the liquid surface is below the detection position, the control unit 5 can detect that the space between the probe 21 and the pressuring plate 3 is not filled with a predetermined amount of the matching liquid. When it is confirmed that the matching liquid is filled in the tank 1 (YES in S905), the control unit 5 proceeds to step S907 and performs monitoring of the matching liquid in the tank 1 and the probe 21. This processing is processing for abnormality detection for the circulation system that can be performed not only at a measurement preparation stage but also periodically even during measurement or according to an instruction of the user.

On the other hand, when it is confirmed that the liquid surface of the matching liquid in the tank 1 is below the detection position of the liquid-surface detecting unit 11 (NO in S905), the control unit 5 causes the display unit 7 to display an error (S906). The user who confirms the error checks whether the matching liquid leaks in the apparatus and, after taking measures according to necessity, fills the matching liquid and performs the pre-measurement preparation again. As a result of performing the pre-measurement preparation again, when it is detected that the matching liquid is filled in the space, the pre-measurement preparation ends (S908).

The control unit 5 monitors the matching liquid in the tank 1 and between the probe 21 and the pressuring plate 3 even during measurement. Specifically, the control unit 5 performs the processing shown in Fig. 10. After the monitoring processing for the matching liquid is started in step S1001, liquid surface detection in the tank 1 in step S1002 and processing performed when the liquid surface is below the detection position (S1003 to S1005) are the same as the processing of the flow shown in Fig. 6.

On the other hand, when the liquid surface in the tank 1 is above the detection position (YES in S1002), in this embodiment, the control unit 5 proceeds to step S1006 and detects the matching liquid surface in the probe 21. The control unit 5 continuously performs the measurement while it is detected that the matching liquid is filled in the space between the probe 21 and the pressuring plate 3. That is, when the matching liquid surface in the probe 21 is above the detection position (YES in S1006), the control unit 5 proceeds to step S1009 and ends the processing. Thereafter, the control unit 5 starts the monitoring processing again after a fixed time.

However, when it is detected that the matching liquid is not filled in the space between the probe 21 and the pressuring plate 3 (NO in S1006), the control unit 5 displays an error on the display unit 7 and stops the measurement (steps S1007 and S1008). Consequently, an inaccurate image is not generated in a state in which the matching liquid is not supplied.

The user who confirms the error display by the display unit 7 checks whether the matching liquid leaks in the apparatus and, after taking measures according to necessity, fills the matching liquid and performs the pre-measurement preparation again. By monitoring the position of of the matching liquid surface in the tank 1, it is possible to quickly detect a matching liquid leak in the apparatus.

With the configuration of this embodiment explained above, it is possible to cope with an abnormality of the circulation system for the matching liquid. That is, it is possible to prevent the matching liquid from overflowing the collection tray and, if a leak of the matching liquid occurs, detect the leak and suppress contamination of the apparatus. Further, since the matching-liquid detecting unit is provided in the probe unit, it is possible to perform stable image acquisition. It is possible to acquire a satisfactory image of the object by detecting an abnormality and feeding back the abnormality in this way.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

The present invention employs an object information acquiring apparatus that includes a plate configured to hold an object, a probe unit including a probe configured to receive an acoustic wave generated from the object, a tank configured to store matching liquid for acoustically matching the plate and the probe, a supplying unit configured to supply the matching liquid in the tank to a space between the probe unit and the plate, and a generating unit configured to generate property information of an inside of the object on the basis of the acoustic wave. The tank includes an aperture in a portion facing the plate, and the volume of the tank is larger than a sum of the internal volume of the supplying unit and the volume of the space between the plate and the probe unit.

## Claims

1. An object information acquiring apparatus comprising:

   a plate configured to hold an object;
   a probe unit including a probe configured to receive, via the plate, an acoustic wave generated from the object;
   a tank located below the plate and configured to store matching liquid for acoustically matching the plate and the probe;
   a supplying unit configured to supply the matching liquid in the tank to a space between the probe unit and the plate; and
   a generating unit configured to generate property information of an inside of the object on the basis of the acoustic wave, wherein
   the tank includes an aperture in a portion facing the plate, and a volume of the tank is larger than a sum of an internal volume of the supplying unit and a volume of the space between the plate and the probe unit.

2. The object information acquiring apparatus according to claim 1, wherein the supplying unit includes a pump configured to deliver the matching liquid and a pipe for connecting the pump to the tank and the probe unit.

3. The object information acquiring apparatus according to claim 1 or 2, further comprising a moving unit configured to move the probe unit along the plate, wherein
   the tank collects the matching liquid flowing from above.

4. The object information acquiring apparatus according to any one of claims 1 to 3, further comprising a detecting unit configured to detect an amount of the matching liquid in the tank.

5. The object information acquiring apparatus according to claim 4, wherein, when the amount of the matching liquid in the tank is smaller than a predetermined amount, the supplying unit stops the supply of the matching liquid.

6. The object information acquiring apparatus according to claim 4 or 5, further comprising a cover configured to cover a region above the detecting unit.

7. The object information acquiring apparatus according to any one of claims 1 to 6, further comprising a second detecting unit configured to detect an amount of the matching liquid accumulating in the space between the probe unit and the plate.

8. The object information acquiring apparatus according to claim 7, wherein, when the amount of the matching liquid accumulating in the space between the probe unit and the plate is smaller than a second predetermined amount, the supplying unit performs the supply of the matching liquid until the second predetermined amount is reached.

FIG. 1

OPENING

OBJECT
SUPPORTING PLATE

FIG. 2A

FIG. 2B

EP 2 636 362 A1

FIG. 3

FIG. 4

S501 —— ( START MEASUREMENT PREPARATION )

S502 —— ◇ MATCHING LIQUID SURFACE IN TANK IS ABOVE DETECTION POSITION ? ◇  NO

S503

YES

DISPLAY ERROR ON DISPLAY UNIT

S504 —— | DRIVE PUMP |

S505 —— | MONITOR MATCHING LIQUID |

S506 —— ( END )

# FIG. 5

S601 — START MATCHING LIQUID MONITORING

S602 — MATCHING LIQUID SURFACE IN TANK IS ABOVE DETECTION POSITION ?

YES

NO

S603 — STOP MEASUREMENT

S604 — STOP PUMP

S605 — DISPLAY ERROR ON DISPLAY UNIT

S606 — END

# FIG. 6

FIG. 7

FIG. 8

S901 — START MEASUREMENT PREPARATION

S902 — MATCHING LIQUID SURFACE IN TANK IS ABOVE DETECTION POSITION ?

NO → DISPLAY ERROR ON DISPLAY UNIT — S903

YES

S904 — DRIVE PUMP

DISPLAY ERROR ON DISPLAY UNIT — S906

S905 — MATCHING LIQUID SURFACE IN PROBE IS ABOVE DETECTION POSITION ?

NO

YES

S907 — MATCHING LIQUID MONITORING IN TANK AND IN PROBE

S908 — END

FIG. 9

S1001 — START MATCHING LIQUID MONITORING

S1002 — MATCHING LIQUID SURFACE IN TANK IS ABOVE DETECTION POSITION ?

NO

YES

S1003 — STOP PUMP

S1004 — STOP MEASUREMENT

S1005 — DISPLAY ERROR ON DISPLAY UNIT

S1006 — MATCHING LIQUID SURFACE IN PROBE IS ABOVE DETECTION POSITION ?

YES

NO

S1007 — STOP MEASUREMENT

S1008 — DISPLAY ERROR ON DISPLAY UNIT

S1009 — END

# FIG. 10

FIG. 11

OPENING

LIQUID SURFACE
SENSOR

ACOUSTIC WAVE
CONVERTER

PROBE

SEAL
MATERIAL

COLLECTION PORT

DELIVERY PORT

# FIG. 12

Europäisches
Patentamt

European
Patent Office

Office européen
des brevets

**EUROPEAN SEARCH REPORT**

Application Number

EP 13 15 7944

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2011/058724 A1 (CANON KK [JP]; FUJIMOTO AKIRA [JP]; TOKITA TOSHINOBU [JP]) 19 May 2011 (2011-05-19) * paragraphs [0016], [0020], [0021], [0029] * * figures 1,2 * | 1-8 | INV. A61B5/00 |
| A | WO 00/09014 A1 (RICHTER KARI [DE]) 24 February 2000 (2000-02-24) * figure 12 * | 1-8 | |

TECHNICAL FIELDS
SEARCHED        (IPC)

A61B

The present search report has been drawn up for all claims

| Place of search | Date of completion of the search | Examiner |
|---|---|---|
| The Hague | 7 June 2013 | Worms, Georg |

EPO FORM 1503 03.82 (P04C01)

**ANNEX TO THE EUROPEAN SEARCH REPORT
ON EUROPEAN PATENT APPLICATION NO.**

EP 13 15 7944

This annex lists the patent family members relating to the patent documents cited in the above-mentioned European search report.
The members are as contained in the European Patent Office EDP file on
The European Patent Office is in no way liable for these particulars which are merely given for the purpose of information.

07-06-2013

| Patent document cited in search report | | Publication date | Patent family member(s) | | Publication date |
|---|---|---|---|---|---|
| WO 2011058724 | A1 | 19-05-2011 | CN | 102596053 A | 18-07-2012 |
| | | | EP | 2498682 A1 | 19-09-2012 |
| | | | JP | 2011103913 A | 02-06-2011 |
| | | | US | 2012150012 A1 | 14-06-2012 |
| | | | WO | 2011058724 A1 | 19-05-2011 |
| WO 0009014 | A1 | 24-02-2000 | NONE | | |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• JP 2011103913 A